# EUROPEAN PATENT APPLICATION

(11) **EP 4 071 457 A1**
(43) Date of publication of application: **12.10.2022**
(21) Application number: 20903564.1
(22) Date of filing: 16.11.2020
(51) Int. Cl.: G01N 15/06

(54) **METHOD FOR MEASURING CONCENTRATION OF MICRO/NANO PARTICLES**

(30) Priority: 19.12.2019 CN 201911319390
(71) Applicant: Resun (Shenzhen) Tech Co., Ltd., Shenzhen, Guangdong 518110 (CN)
(72) Inventor: XIONG, Gui, Shenzhen, Guangdong 518110 (CN); LIU, Ke, Shenzhen, Guangdong 518110 (CN); WANG, Zhe, Shenzhen, Guangdong 518110 (CN)
(74) Representative: Manitz Finsterwald Patent- und Rechtsanwaltspartnerschaft mbB
(86) International application number: PCT/CN2020/128991
(87) International publication number: WO 2021/120944

(57) **Abstract**

A method for measuring the concentration of a micro/nano particle, comprising: allowing the to-be-measured micro/nano particle to bind with one or more kinds of marker to form a new particle, the new particle having a change in at least one of particle size, charge state, and particle morphology compared with the to-be-measured micro/nano particle or the marker; measuring the particle size, charge state, or particle morphology of the new particle and the to-be-measured micro/nano particle or the marker, and counting the new particle and the to-be-measured micro/nano particle or the marker respectively to obtain their respective count results, and, on the basis of the count results, calculating the concentration of the to-be-measured micro/nano particle bound with the marker. The method of the present application has the advantages of high measurement accuracy, low measurement limit, and stability of chemical reagents.

## Description

### Technical Field

The present application relates to the technical field of particle measurement, in particular to a method for measuring the concentration of a micro/nano particle.

### Background Art

The concentration of particles in a liquid is such an important physical parameter that ranges of concentration of particles are specifically set out in industrial standards related to pharmaceuticals, semiconductors, coatings and inks, filtration, etc. Measurement of particle concentration is a very important fundamental task, and is widely employed for cement, ceramics, pharmaceuticals, emulsions, coatings, dyes, pigments, fillers, chemical products, catalysts, drilling mud, abrasives, lubricants, coal powder, silt, dust, cells, bacteria, foods, additives, pesticides, explosives, graphite, photosensitive materials, fuels, inks, metal and non-metal powders, calcium carbonate, kaolin, coal-water slurry and other powdery materials.

There are a number of methods for measuring the concentration of micro/nano particles. The impedance-based measurement technique has the distinct advantage of enabling efficient identification of the characteristics of a single particle. However, the Coulter principle underlying the impedance-based method can only allow measurement of an equivalent particle size of particles, and fails to allow measurement of the potential and morphology of the particles. Moreover, the impedance-based method has a low precision in the measurement of small-sized particles.

The colorimetric method is based on the principle of the Lambert-Beer law that the absorbance of light by suspended particles in a solution is proportional to their concentration in the solution. However, the accuracy of this method is limited by intensity of light absorption. It is relatively accurate when the absorbance is in the range of from 1 to 2, and cannot measure particles having a relatively low concentration.

Both the fluorescence method and the chemiluminescence method achieve measurement of target particles by labeling the target particles with a specific light-emitting group and measuring the intensity of light emission. They suffer from relatively high costs of measurement devices on the one hand, and the problem of stability of the chemical reagents having a light-emitting group.

### Summary of the Disclosure

The present application provides a novel method for measuring the concentration of a micro/nano particle on the basis of individual particles. The method has the advantages of high measurement accuracy, low measurement limit, and stability of chemical reagents.

According to a first aspect of the present application, there is provided a method for measuring the concentration of a micro/nano particle, the method comprising:
allowing the to-be-measured micro/nano particle to bind with one or more kinds of marker to form a new particle, the new particle having a change in at least one of particle size, charge state, and particle morphology compared with the to-be-measured micro/nano particle or the marker;
measuring the particle size, charge state, or particle morphology of the new particle and the to-be-measured micro/nano particle or the marker, and counting the new particle and the to-be-measured micro/nano particle or the marker respectively to obtain their respective count results, and, on the basis of the count results, calculating the concentration of the to-be-measured micro/nano particle bound with the marker.

In preferred embodiments, the marker is a polystyrene microsphere, a magnetic bead, a silica sphere or a micelle that carries a specific functional group or a specific antigen or antibody.

In preferred embodiments, the specific functional group is selected from a biological enzyme, a substrate, an antigen, an antibody, a ligand, a receptor, biotin, and avidin.

In preferred embodiments, the method in which the to-be-measured micro/nano particle binds with the marker includes specific antigen-antibody binding, a special immunochemical reaction, a specific chemical synthesis reaction, and specific binding of an aptamer.

In preferred embodiments, the way of binding of the to-be-measured micro/nano particle with the marker includes binding of one to-be-measured micro/nano particle with one marker, binding of one to-be-measured micro/nano particle with a plurality of markers, and binding of a plurality of to-be-measured micro/nano particles with one marker.

In preferred embodiments, the to-be-measured micro/nano particle is selected from a protein, an exosome, or a virus.

In preferred embodiments, methods for measuring the particle size, charge state, or particle morphology of the new particle and the to-be-measured micro/nano particle or the marker include nanoparticle tracking analysis (NTA), tunable resistive pulse sensing (TRPS), single particle pulse technique and microscopic imaging method.

According to a second aspect of the present application, there is provided a method for purifying a micro/nano particle and measuring a concentration thereof, the method comprising:
allowing a sample comprising the to-be-measured micro/nano particle to specifically bind with one or more kinds of magnetic bead marker in a solution, the magnetic beads carrying an antigen, an antibody, an aptamer or a specific functional group that specifically binds to the to-be-measured micro/nano particle, such that a conjugate resulting from the binding has a change in at least one of particle size, charge state, and particle morphology compared with the to-be-measured micro/nano particle or the magnetic bead marker;
placing the mixed solution formed in the previous step in a magnetic field, such that the magnetic beads are magnetically adsorbed at a local region of the magnetic field, and then adding a cleaning liquid to replace the mixed solution in the region of the magnetic field, such that particles other than the to-be-measured micro/nano particle in the sample are removed along with replacement of the mixed solution, so that the cleaning liquid in the region of the magnetic field contains only the magnetic bead particles;
measuring a change in the particle size, charge state, or particle morphology of the magnetic beads bound with the to-be-measured micro/nano particle in comparison to the magnetic beads not bound with the to-be-measured micro/nano particle in the cleaning liquid obtained in the previous step, respectively counting the magnetic beads bound with the to-be-measured micro/nano particle and the magnetic beads not bound with the to-be-measured micro/nano particle to obtain their respective count results, and, on the basis of the count results,
calculating the concentration of the to-be-measured micro/nano particle bound with the magnetic beads.

In preferred embodiments, the cleaning liquid is a liquid that does not comprise any particles that interfere with detection of the magnetic beads.

In preferred embodiments, the to-be-measured micro/nano particle is selected from a protein, an exosome, or a virus.

The method for measuring the concentration of a micro/nano particle according to the present application achieves measurement, on the basis of individual particles, by allowing the to-be-measured micro/nano particle to bind with a marker to form a new particle, measuring a change in the particle size, charge state, or particle morphology of the new particle and the to-be-measured micro/nano particle or the marker, and counting the new particle and the to-be-measured micro/nano particle or the marker respectively so as to obtain the concentration of the to-be-measured micro/nano particle bound with the marker. The method has the advantages of high measurement accuracy, low measurement limit, and stability of chemical reagents.

### Brief Description of the Drawings

Fig. 1 is a bar graph of particle size distribution in Example 1 of the present application;
Fig. 2 is a scatter diagram of particle size v.s. Zeta potential distribution in Example 2 of the present application;
Fig. 3 is a scatter diagram of magnetic bead particle size v.s. length-to-diameter ratio in Example 3 of the present application; and
Fig. 4 is a scatter diagram of particle size v.s Zeta potential distribution in Example 4 of the present application.

### Detailed Description of the Embodiments

The present application will be further illustrated below by detailed description of embodiments with reference to the accompanying drawings. In the following embodiments, many details are described so that the present application will be better understood. However, those skilled in the art can readily recognize that some of the features may be omitted, or replaced by other materials or methods, depending on different situations.

Additionally, the characteristics, operations or features described in the specification can be combined in any suitable manner to form various embodiments. Moreover, the steps or actions in the description of the method may also be switched or adjusted in sequence in a manner that is obvious to those skilled in the art. Therefore, the various sequences in the description and the drawings are merely for the purpose of clearly describing a particular embodiment and are not intended to be required, unless it is otherwise specified that a specific sequence must be followed.

In an aim to address the disadvantages of high costs, high detection limit and low accuracy in the measurement of micro/nano particles in the prior art, the present application provides a novel method for measuring the concentration of a micro/nano particle, in which the to-be-measured micro/nano particle (the target particle) is allowed to specifically bind to a marker, and counting is performed on the basis of a marked change in the particle size, charge state, or particle morphology so as to obtain the concentration of the target particle.

The present application is directed to various micro/nano particles, such as proteins, exosomes, viruses, etc., which cannot be directly counted, and which can be measured and analyzed by using the method of the present application. For example, in embodiments of the present application, the micro/nano particles measured are exosomes (e.g., kidney cell exosomes or lung tumor cell exosomes), Tau protein, Troponin I, etc. These micro/nano particles can come from various tissues or body fluids of various organisms (such as human body), for example, kidney cell exosomes from urine, Tau protein from cerebrospinal fluid, Troponin I from blood, or lung tumor cell exosomes from blood, etc.

The present application enables measurement of particles that vary in size over a wide range. Particles ranging from nano-scale to micro-scale can all be measured using the method of the present application, with particles having a size in the range of from 5 nm to 5 µm being preferably measured.

According to an embodiment of the present aplication, a method for measuring the concentration of a micro/nano particle comprises:
allowing the to-be-measured micro/nano particle to bind with one or more kinds of marker to form a new particle, the new particle having a change in at least one of particle size, charge state, and particle morphology compared with the to-be-measured micro/nano particle or the marker;
measuring the particle size, charge state, or particle morphology of the new particle and the to-be-measured micro/nano particle or the marker, and counting the new particle and the to-be-measured micro/nano particle or the marker respectively to obtain their respective count results, and, on the basis of the count results, calculating the concentration of the to-be-measured micro/nano particle bound with the marker.

In embodiments of the present application, the marker is a substance that can bind to the to-be-measured micro/nano particle and cause a change in one or more features of particle size, charge state, and particle morphology of the to-be-measured micro/nano particle. Such a marker can be a polystyrene microsphere, a magnetic bead, a silica sphere or a micelle that carries a specific functional group or a specific antigen or antibody. The specific functional group can be selected from such compounds or groups as a biological enzyme, a substrate, an antigen, an antibody, a ligand, a receptor, biotin, and avidin.

In embodiments of the present application, generally, the marker is also a particle of nano-scale or micro-scale. For example, in an embodiment of the present application, the marker is a polystyrene microsphere carrying an antibody capable of specific binding. The polystyrene microsphere has a particle size of hundreds of nanometers, such as, 100 nm, 150 nm, 200 nm, 300 nm, 400 nm, 500 nm, etc., and preferably 300 nm, and can have a concentration, as counted individually in a measurement solution, of 1 × 10¹⁰/ml to 1 × 10⁷/ml, preferably 1 × 10⁹/ml. In another embodiment of the present application, the marker is a silica microsphere carrying an antibody capable of specific binding. The silica microsphere has a particle size of hundreds of nanometers, such as, 100 nm, 150 nm, 200 nm, 250 nm, 300 nm, 350 nm, 400 nm, 450 nm, 500 nm, etc., and preferably 200 nm, and can have a concentration, as counted individually in a measurement solution, of 1 × 10¹⁰/ml to 1 × 10⁷/ml, preferably 1 × 10⁸/ml.In yet another embodiment of the present application, the marker is a magnetic bead carrying an antibody capable of specific binding. The magnetic bead has a particle size of tens to hundreds of nanometers, such as, 30 nm, 50 nm, 80 nm, 100 nm, 150 nm, 200 nm, 300 nm, 400 nm, 500 nm, etc., and preferably 100 nm, and can have a concentration, as counted individually in a measurement solution, of 1 × 10¹⁰/ml to 1 × 10⁷/ml, preferably 1 × 10⁸/ml.

In embodiments of the present application, methods in which the to-be-measured micro/nano particle binds with the marker include specific antigen-antibody binding, a special immunochemical reaction, a specific chemical synthesis reaction, and specific binding of an aptamer.

In embodiments of the present application, ways of binding of the to-be-measured micro/nano particle with the marker include binding of one to-be-measured micro/nano particle with one marker, binding of one to-be-measured micro/nano particle with a plurality of markers, and binding of a plurality of to-be-measured micro/nano particles with one marker.

In embodiments of the present application, the new particle formed from binding of the marker to the to-be-measured micro/nano particle has a change in at least one features of particle size, charge state, and particle morphology compared with the marker or the to-be-measured micro/nano particles. The indicator to measure the size of a particle is generally particle size, that is, the diameter of the particle, and, in particular, average particle size; the indicator to measure the charge state is generally the amount of charge or electromobility (that is, the migration rate in electrophoresis); and the particle morphology can be, for example, spherical, ellipsoidal or other irregular shapes.

In embodiments of the present application, a number of method are available for measuring the particle size, charge state, or particle morphology of the new particle and the to-be-measured micro/nano particle or the marker, typical but not limitative examples including nanoparticle tracking analysis (NTA), tunable resistive pulse sensing (TRPS), single particle pulse technique and microscopic imaging method. For example, in an embodiment of the present application, the nanoparticle tracking analysis (NTA) technique is used to measure the particle size and number of polystyrene microspheres and the new particles formed after binding of exosomes to the polystyrene microspheres. In another embodiment of the present application, the tunable resistive pulse sensing (TRPS) technique is used to measure the particle size and number of silica microspheres and the new particles formed after binding of Tau protein to the silica microspheres.In yet another embodiment of the present application, the single particle pulse technique is used to measure the length-to-diameter ratio and number of nano magnetic beads and the new particles formed after binding of Troponin I to the nano magnetic beads. In still another embodiment of the present application, the single particle pulse technique is used to measure the particle size and charge of lung tumor cell exosomes in a blood sample before and after binding with a specific antibody and to obtain a scatter diagram of particle size v.s. Zeta distribution.

The present application enables calculation of the concentration of a to-be-measured micro/nano particle bound with a marker on the basis of count results of the new particle and the to-be-measured micro/nano particle or the marker. The result of calculation is related to the count results of the respective kinds of particle and the reaction pattern. For example, in an embodiment of the present application, a polystyrene microsphere (as a marker) and an exosome (as a to-be-measured micro/nano particle) bind to each other in a one-to-one manner. The concentration of the polystyrene microsphere is designated as n, the number of the polystyrene microsphere not bound with the exosome is designated as N1, and the number of polystyrene microsphere bound with the exosome is designated as N2, then the number of the exosome in a to-be-measured solution is: n^{∗}N2/(N1+N2). In another embodiment of the present application, a silica microsphere (as a marker) and a Tau protein (as a to-be-measured micro/nano particle) bind to each other in a two-to-one manner. The concentration of the silica microsphere is designated as n, the number of the silica microsphere not bound with the Tau protein is designated as N1, and the number of silica microsphere bound with the Tau protein is designated as N2, then the number of the Tau protein in a to-be-measured solution is: 2^{∗}n^{∗}N2/(N1+2^{∗}N2).

Another embodiment of the present application provides a method for purifying a micro/nano particle and measuring the concentration thereof, the method comprising comprising:
allowing a sample comprising the to-be-measured micro/nano particle to specifically bind with one or more kinds of magnetic bead marker in a solution, the magnetic beads carrying an antigen, an antibody, an aptamer or a specific functional group that specifically binds to the to-be-measured micro/nano particle, such that a conjugate resulting from the binding has a change in at least one of particle size, charge state, and particle morphology compared with the to-be-measured micro/nano particle or the magnetic bead marker;
placing the mixed solution formed in the previous step in a magnetic field, such that the magnetic beads are magnetically adsorbed at a local region of the magnetic field, and then adding a cleaning liquid to replace the mixed solution in the region of the magnetic field, such that particles other than the to-be-measured micro/nano particle in the sample are removed along with the mixed solution, so that the cleaning liquid in the region of the magnetic field contains only the magnetic bead particles;
measuring a change in the particle size, charge state, or particle morphology of the magnetic beads bound with the to-be-measured micro/nano particle in comparison to the magnetic beads not bound with the to-be-measured micro/nano particle in the cleaning liquid obtained in the previous step, respectively counting the magnetic beads bound with the to-be-measured micro/nano particle and the magnetic beads not bound with the to-be-measured micro/nano particle to obtain their respective count results, and, on the basis of the count results, calculating the concentration of the to-be-measured micro/nano particle bound with the magnetic beads.

In embodiments of the present application, the cleaning liquid used to clean the magnetic beads needs to be free of interference with the measurement of the magnetic bead particles as much as possible, for example, free of any magnetic particles contained therein.In an embodiment of the present application, the cleaning liquid used to clean the magnetic beads is a liquid that does not contain particles that interferes with detection of the magnetic beads, for example, physiological saline cleaning liquid.

The method for measuring the concentration of a micro/nano particle according to the present application achieves measurement, on the basis of individual particles, by allowing the to-be-measured micro/nano particle to bind with a marker to form a new particle, measuring a change in the particle size, charge state, or particle morphology of the new particle and the to-be-measured micro/nano particle or the marker, and counting the new particle and the to-be-measured micro/nano particle or the marker respectively so as to obtain the concentration of the to-be-measured micro/nano particle bound with the marker. The method has the advantages of high measurement accuracy, low measurement limit, and stability of chemical reagents.

The technical solution of the present application will be described in detail below by way of specific examples. It is appreciated that the examples are merely illustrative and should not be construed as limiting the scope of protection of the present application.

### Example 1

Measuring the concentration of kidney cell exosomes in urine using polystyrene microspheres carrying an antibody capable of specific binding

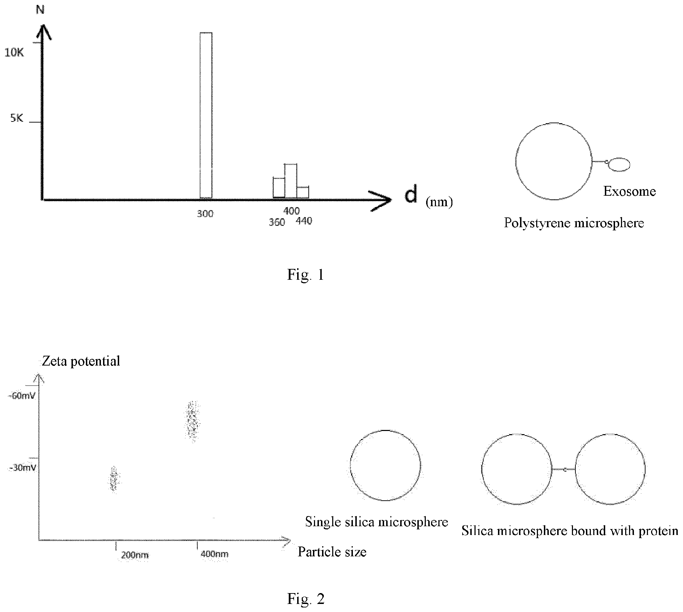

A urine sample from a patient with nephritis was centrifuged using an ultracentrifuge. Supernatant was removed, and mixed 1:1 with a reagent solution containing antibody-modified polystyrene microspheres (particle size being 300 nm, and concentration being 1×10⁹/ml), the polystyrene microspheres carrying an antibody capable of specific binding to a surface membrane protein of kidney cell exosomes. The mixed solution was allowed to react at 37°C for 20 minutes. Then the mixed solution was measured using the NTA (nanoparticle tracking analysis) technique to achieve the measurement of the particle size of the polystyrene microspheres. A bar graph of the particle size distribution as shown in Fig. 1 was obtained.

It was determined that the number N1 of particles having a particle size in the range of 300±20 nm was 11254, the number of particles within this range being the number of polystyrene microspheres not bound with the exosomes; and the number N2 of particles in the range of 340 nm to 460 nm was 2430, the number of particles within this range being the number of polystyrene microspheres bound with the exosomes.

The number of kidney cell exosomes in the original sample was: n^{∗}N2/(N1+N2)=1.77×10⁸/ml.

### Example 2

Measuring the concentration of Tau protein in cerebrospinal fluid using silica microspheres carrying an antibody capable of specific binding

A cerebrospinal fluid sample from a patient with Alzheimer's disease was filtered using a porous resin. The solution obtained after filtration was mixed 1:1 with a reagent solution containing two kinds of antibody-modified silica microspheres (particle size both being 200 nm, concentration n both being 1×10⁸/ml, and Zeta potential being -25 mV), one kind of silica microspheres carrying an antibody capable of specific binding to a binding site of Tau protein, and the other kind of silica microspheres carrying an antibody capable of specific binding to another binding site of Tau protein. The mixed solution was allowed to react at 37°C for 20 minutes. Then the mixed solution was measured using the TRPS (tunable resistive pulse sensing) technique to achieve the measurement of the particle size and charge of the silica microspheres. A scatter diagram of particle size v.s. Zeta potential distribution as shown in Fig. 2 was obtained.

It was determined that the number N1 of particles having a particle size in the range of 200±5 nm and having a Zeta potential of around -25mV was 5434, the number of particles within this range being the number of silica microspheres not bound with Tau protein; and the number N2 of particles in the range of 380±10 nm was 630, the number of particles within this range being the number of silica microspheres bound with Tau protein through either antibody.

The number of Tau protein in the original sample was: 2^{∗}n^{∗}N2/(N1+2^{∗}N2)=1.88×10⁷/ml.

### Example 3

Measuring the concentration of Troponin I in blood using magnetic beads carrying an antibody capable of specific binding

A blood sample from a patient with heart disease was mixed 1:1 with a reagent solution containing two kinds of antibody-modified magnetic beads, one kind of magnetic beads carrying an antibody capable of specific binding to a binding site of Troponin I (cTnI), having a particle size of 100 nm and having a concentration n of 1×10⁸/ml, and the other kind of magnetic beads carrying an antibody capable of specific binding to another binding site of Troponin I (cTnI), having a particle size of 200 nm and having a concentration n of 1×10⁸/ml. The mixed solution was allowed to react at 37°C for 20 minutes.

The mixed solution was poured into a test tube. The test tube was placed in a strong magnetic field such that the magnetic beads were enriched at a lower part of the test tube. Liquid at an upper part of the test tube was removed using a pipette, and the magnetic field was removed. Physiological saline was added to the test tube, and the content was mixed well. The test tube was again placed in a strong magnetic field. The above steps were repeated until the physiological saline in the test tube contained only high purity magnetic beads.

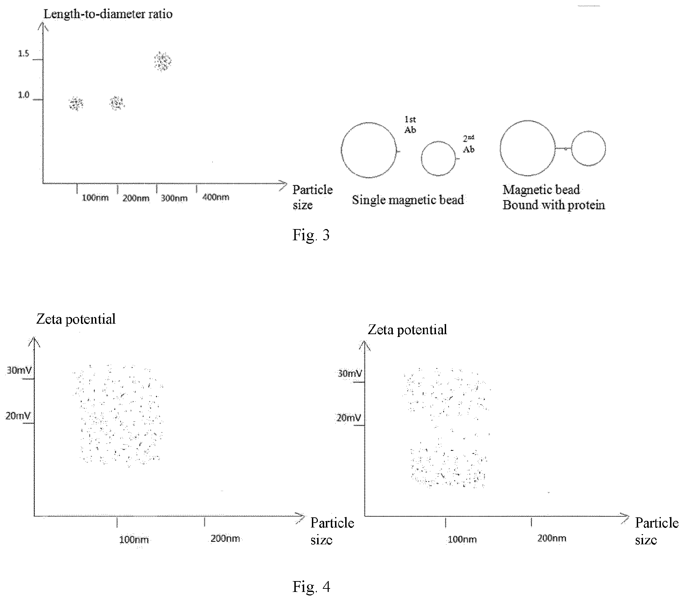

The above physiological saline containing magnetic beads was measured using the single particle pulse technique, and a scatter diagram of magnetic bead particle size v.s. length-to-diameter ratio as shown in Fig. 3 was obtained.

It was determined that the number N1 of particles having a particle size in the range of 200±5 nm and having a length-to-diamater ratio of around 1.0 was 4246, the number N2 of particles having a particle size in the range of 100±5 nm and having a length-to-diamater ratio of around 1.0 was 4240, the number of particles within these ranges being the number of magnetic beads not bound with Troponin I; and the number N3 of particles having a particle size in the range of 290±10 nm and having a length-to-diamater ratio of around 1.5 was 543, the number of particles within this range being the number of magnetic beads bound with Troponin I through either antibody.

The number of Troponin I in the original sample was: 2^{∗}n^{∗}N3/(N1+N2+2^{∗}N3)=1.13×10⁷/ml.

### Example 4

Measuring the concentration of lung tumor cell exosomes in blood using an antibody capable of specific binding

A blood sample from a patient with lung tumor was centrifuged using an ultracentrifuge. Supernatant was removed, which contained no large particles such as cells, platelets, etc. A portion of the supernatant was measured using the single particle pulse technique to achieve the measurement of the particle size and charge of exosomes. A scatter diagram of particle size v.s. Zeta potential distribution as shown in the left panel of Fig. 4 was obtained.

It was determined that the total number N1 of the exosomes was 4230, the total concentration n of the exosomes was 5.34×10⁸/ml, and the number N2 of the exosomes having a Zeta potential of lower than 20 mV was 1014.

To the supernatant was added a reagent solution containing an antibody to a surface membrane protein of lung tumor cell exosomes (the antibody being in an excess amount in the reagent, and the antibody having a negative Zeta potential). The mixed solution was allowed to react at 37°C for 20 minutes. Then the mixed solution was measured using the single particle pulse technique to achieve the measurement of the particle size and charge of the exosomes. A scatter diagram of particle size v.s. Zeta potential distribution as shown in the right panel of Fig. 4 was obtained.The lung tumor cell exosomes had decreased charge after binding to the antibody. It was determined that the total number M1 of the exosomes was 3802, and the number M2 of the exosomes having a Zeta potential of lower than 20 mV was 2011, with the increased number of the exosomes having a Zeta potential of lower than 20 mV being the number of lung cell exosomes.

The number of lung cell exosomes in the original sample was: n^{∗}(M2/M1-N2/N1)=1.54×10⁸/ml.

The present invention has been described above with reference to specific examples, which are merely intended to aid the understanding of the present invention and are not intended to limit the present invention thereto. Several simple derivations, variations or substitutions can be made by a person skilled in the art to which the present invention pertains in light of the concept of the present invention.

## Claims

1. A method for measuring the concentration of a micro/nano particle, the method comprising:
allowing the to-be-measured micro/nano particle to bind with one or more kinds of marker to form a new particle, the new particle having a change in at least one of particle size, charge state, and particle morphology compared with the to-be-measured micro/nano particle or the marker;
measuring the particle size, charge state, or particle morphology of the new particle and the to-be-measured micro/nano particle or the marker, and counting the new particle and the to-be-measured micro/nano particle or the marker respectively to obtain their respective count results, and, on the basis of the count results, calculating the concentration of the to-be-measured micro/nano particle bound with the marker.

2. The method according to claim 1, wherein the marker is a polystyrene microsphere, a magnetic bead, a silica sphere or a micelle that carries a specific functional group or a specific antigen or antibody.

3. The method according to claim 2, wherein the specific functional group is selected from a biological enzyme, a substrate, an antigen, an antibody, a ligand, a receptor, biotin, and avidin.

4. The method according to claim 1, wherein methods in which the to-be-measured micro/nano particle binds with the marker include specific antigen-antibody binding, a special immunochemical reaction, a specific chemical synthesis reaction, and specific binding of an aptamer.

5. The method according to claim 1, wherein ways of binding of the to-be-measured micro/nano particle with the marker include binding of one to-be-measured micro/nano particle with one marker, binding of one to-be-measured micro/nano particle with a plurality of markers, and binding of a plurality of to-be-measured micro/nano particles with one marker.

6. The method according to claim 1, wherein the to-be-measured micro/nano particle is selected from a protein, an exosome, a virus, a polystyrene microsphere, a magnetic bead, a silica sphere or a micelle.

7. The method according to claim 1, wherein methods for measuring the particle size, charge state, or particle morphology of the new particle and the to-be-measured micro/nano particle or the marker include nanoparticle tracking analysis (NTA), tunable resistive pulse sensing (TRPS), single particle pulse technique and microscopic imaging method.

8. A method for purifying a micro/nano particle and measuring a concentration thereof, the method comprising:
allowing a sample comprising the to-be-measured micro/nano particle to specifically bind with one or more kinds of magnetic bead marker in a solution, the magnetic beads carrying an antigen, an antibody, an aptamer or a specific functional group that specifically binds to the to-be-measured micro/nano particle, such that a conjugate resulting from the binding has a change in at least one of particle size, charge state, and particle morphology compared with the to-be-measured micro/nano particle or the magnetic bead marker;
placing the mixed solution formed in the previous step in a magnetic field, such that the magnetic beads are magnetically adsorbed at a local region of the magnetic field, and then adding a cleaning liquid to replace the mixed solution in the region of the magnetic field, such that particles other than the to-be-measured micro/nano particle in the sample are removed along with the mixed solution, so that the cleaning liquid in the region of the magnetic field contains only the magnetic bead particles;
measuring a change in the particle size, charge state, or particle morphology of the magnetic beads bound with the to-be-measured micro/nano particle in comparison to the magnetic beads not bound with the to-be-measured micro/nano particle in the cleaning liquid obtained in the previous step, respectively counting the magnetic beads bound with the to-be-measured micro/nano particle and the magnetic beads not bound with the to-be-measured micro/nano particle to obtain their respective count results, and, on the basis of the count results, calculating the concentration of the to-be-measured micro/nano particle bound with the magnetic beads.

9. The method according to claim 8, wherein the cleaning liquid is a liquid that does not comprise any particles that interfere with detection of the magnetic beads.

10. The method according to claim 8, wherein the to-be-measured micro/nano particle is selected from a protein, an exosome, a virus, a polystyrene microsphere, a magnetic bead, a silica sphere or a micelle.
